# EUROPÄISCHE PATENTSCHRIFT

(11) **EP 2 404 567 B1**
(45) Veröffentlichungstag und Bekanntmachung des Hinweises auf die Patenterteilung: **27.04.2016**
(21) Anmeldenummer: 11005334.5
(22) Anmeldetag: 30.06.2011
(51) Int. Cl.: A61L 2/26

(54) **Medizinischer Sterilisierbehälter mit Gasaustauschfilter**
Medical sterilisation container with gas filter
Récipient médical de stérilisation comprenant un filtre à échange de gaz

(30) Priorität: 06.07.2010 DE 202010009925 U
(43) Veröffentlichungstag der Anmeldung: 11.01.2012
(73) Patentinhaber: Innovations Medical GmbH, 78532 Tuttlingen (DE)
(72) Erfinder: Kreidler, Winfried, 78532 Tuttlingen (DE)
(74) Vertreter: Binner, Bernhard

(56) Entgegenhaltungen:
- EP-A1- 1 563 854
- WO-A1-03/041749
- WO-A1-2005/099610
- DE-A1- 2 610 290
- Günter Spur,Theodor Stöferle: "Handbuch der Fertigungstechnik: Fügen, Handhaben und Montieren", 31. Dezember 1986 (1986-12-31), XP000002658356, Seite 34,

## Beschreibung

Die Erfindung betrifft einen medizinischen, mit einem abnehmbaren Behälterdeckel versehenen Sterilisierbehälter, der in einem mit Gasaustauschöffnungen versehenen Wandbereich mit einer Filtereinheit für den Gasaustausch versehen ist, die ein austauschbares, die Gasaustauschöffnungen innenseitig abdeckendes Filterblatt aufweist, das innerhalb eines ringförmig umlaufenden Rahmenelements angeordnet und durch eine mit Durchbrüchen versehene Andruckscheibe gegen die Innenseite des Wandbereichs pressbar gehalten ist, wobei die Andruckscheibe lösbar mit dem Rahmenelement oder dem Wandbereich verbunden ist und eine Ringnut mit einem vorzugsweise elastischen Dichtungsring aufweist, der den mit den Gasaustauschöffnungen versehenen Wandbereich gegen das Rahmenelement abdichtet.

EP1563854 A1 offenbart einen medizinischen Sterilisierbehälter mit einem abnehmbaren Deckel, der in einem durchbrochenen Gasaustauschbereich eine Filtereinheit für den Gasaustausch aufweist. Innerhalb eines umlaufenden Rahmenelements ist ein Filterblatt zwischen zwei mit Durchbrüchen versehenen Auflageflächen auswechselbar angeordnet. Ein Halter ist durch mehrere Renkverbindungselemente lösbar mit dem Rahmenelement verbunden. In einer Ringnut ist ein elastischer Dichtungsring angeordnet, der den durchbrochenen Gasaustauschbereich des Deckels gegen das Rahmenelement abdichtet. Das Rahmenelement ist in Form eines dünnwandigen, runden Rings fest und dicht mit der Innenfläche des Deckels verbunden und mit mehreren gleichmässig verteilt angeordneten Renkverschlussbolzen versehen. Der scheibenförmige Halter ist auf der Innenseite eines haubenartig ausgebildeten, mehrfach durchbrochenen Griffteils befestigt, welches im Randbereich des Halters innerhalb eines mit den Renkverschlussbolzen verbindbaren Kupplungsflansches eine Ringnut mit dem Dichtungsring aufweist.

Sterilisierbehälter der gattungsgemäßen Art dienen in der Regel zur Sterilisation von medizinischen Geräten. Hierzu weisen solche Sterilisierbehälter einen Behälterdeckel auf, welcher lösbar mit einem entsprechenden Untergehäuse des Sterilisierbehälter dicht verbunden ist. Um bei geschlossenem Sterilisierbehälter eine "Be- und Entlüftung" sicherzustellen, weist ein solcher Sterilisierbehälter in einem seiner Wandbereiche Gasaustauschöffnungen auf, welche im Betrieb innenseitig mittels eines Filterblattes abgedeckt sind. Ein solches Filterblatt besteht in der Regel aus Papier, einem Vlies oder textilen Werkstoff. Mit zunehmender Betriebsdauer und/oder bei Wechsel der zu sterilisierenden Geräte ist dieses Filterblatt regelmäßig auszutauschen.

Hierzu wird das Filterblatt durch eine Andruckscheibe gehalten, welche mit einem innenseitig am Wandbereich angeordneten, die Gasaustauschöffnungen ringförmig umgebenden Rahmenelement lösbar in Eingriff steht. Dabei wird die lösbare Verbindung zwischen der Andruckscheibe und dem Rahmenelement durch eine Renkverbindung hergestellt, welche aus wechselseitig miteinander in Eingriff bringbaren Renkverschlusselementen bzw. Renkverbindungselementen an der Andruckscheibe einerseits und am Rahmenelement andererseits gebildet wird. Zur Abdichtung der Andruckscheibe gegenüber dem Wandbereich des Sterilisierbehälters bzw. gegenüber dem Rahmenelement ist ein vorzugsweise elastischer Dichtungsring vorgesehen, welcher wandseitig in einer umlaufenden Ringnut der Andruckscheibe aufgenommen wird. Der Dichtungsring drückt im montierten Zustand gegen das Filterblatt, welches vorzugsweise - relativ passgenau - in das ringförmige Rahmenelement eingesetzt ist. Die Druckscheibe, das Filterblatt und das Rahmenelement bilden somit eine Art Filtereinheit.

Durch die zwischen dem Rahmenelement und der Andruckscheibe ausgebildete Renkverbindung kann die Andruckscheibe nur durch eine drehende Relativbewegung aus dem Rahmenelement gelöst und wieder in entgegengesetzter Richtung drehend mit diesem formschlüssig in Eingriff gebracht werden. Da der Dichtungsring der Andruckscheibe dichtend gegen das Filterblatt drückt und die Andruckscheibe dementsprechend unter axialer Vorspannung gegen das Filterblatt insbesondere beim Einbau zu verspannen ist, führt dies regelmäßig zu einer Verschiebung des Filterblattes und/oder zu dessen Beschädigung.

Der Erfindung liegt demgemäß die Aufgabe zugrunde, die Filtereinheit eines Sterilisierbehälters der eingangs genannten Art derart zu verbessern, dass das Filterblatt beim Einspannen nicht verschoben und/oder beschädigt wird.

Gelöst wird diese Aufgabe erfindungsgemäß durch die Merkmale des kennzeichnenden Teils des Anspruch 1.

Durch diese erfindungsgemäße Ausgestaltung der Filtereinheit wird sicher gewährleistet, dass das Filterblatt beim Einspannen nicht verschoben oder beschädigt werden kann, da die das Filterblatt gegen den Wandbereich drückende Andruckscheibe zum Fixieren der Andruckscheibe nicht selbst gedreht werden muss. Sowohl die Federscheibe als auch die Andruckscheibe weisen zumindest auf ihren einander zugewandten Seilen eine glatte Oberfläche auf, so dass die Federscheibe leicht relativ zur Andruckscheibe zur Herstellung der Renkverbindung zum Rahmenelement gedreht werden kann. Damit kann die Federscheibe aber mit einer relativ großen Vorspannung gegen die Andruckscheibe gepresst und gedreht werden, ohne dass sich die Andruckscheibe mitbewegt. Somit wird weiter auch der Vorteil erreicht, dass auf die unmittelbar bzw. mittels eines Dichtungsringes auf dem Filterblatt aufliegende Andruckscheibe ein höherer Anpressdruck ausgeübt wird, wodurch die Dichtwirkung des Dichtungsringes verbessert wird. Solche höheren Anpresskräfte sind bei den bisher bekannten Filtereinheiten nicht möglich, da dort die Andruckscheibe zur Herstellung der Renkverbindung gedreht werden muss und durch den direkten reibschlüssigen Kontakt ihres Dichtungsringes mit dem Filterblatt zur Zerstörung des Filterblattes führen würde.

Weitere vorteilhafte Ausgestaltungen sind den Unteransprüchen zu entnehmen.

So kann gemäß Anspruch 2 vorgesehen sein, dass die Federscheibe mit Durchbrüchen versehen ist, welche im Wesentlichen nach dem gleichen Flächenmuster ausgebildet und angeordnet sind wie die Durchbrüche der Andruckscheibe. Dabei ist vorgesehen, dass die Durchbrüche der Andruckscheibe und der Federscheibe nach der Herstellung der Renkverbindung zwischen der Federscheibe und dem Rahmenelement deckungsgleich übereinander liegen. Dadurch wird ein flächenmäßig größtmöglicher Gasaustausch erreicht.

Gemäß Anspruch 3 kann vorgesehen sein, dass die Renkverbindungselemente der Federscheibe aus an ihrem kreisrunden Umfang radial nach außen vorstehenden nocken- oder zungenartigen Vorsprüngen gebildet sind, die in an der Innenseite des Rahmenelements angeordnete radiale Aufnahmeschlitze oder Nuten eingreifen, welche sich jeweils in einer Drehrichtung an die Einführerweiterungen des Rahmenelements anschließen. Durch diese Anordnung und Ausgestaltung der Renkverbindungselemente lässt sich die Federscheibe einfach und sicher am Rahmenelement des Wandbereichs befestigen und bei Bedarf auch wieder abnehmen. Ein weiterer Vorteil besteht darin, dass sich die Renkverbindungselemente der Federscheibe einfach und leicht herstellen lassen. So kann die gesamte Federscheibe in einem einheitlichen Stanzprozess hergestellt werden, in welchem gleichzeitig sowohl die Außenkontur als auch die Durchbrüche herstellbar sind.

Weiter kann gemäß Anspruch 4 vorgesehen sein, dass sich die Einführerweiterungen über die gesamte Höhe bzw. Dicke des Rahmenelements erstrecken und dass die Andruckscheibe an ihrem Umfang mit radial nach außen vorstehenden, nocken- oder zungenartigen Vorsprüngen versehen ist, die jeweils in einer solchen Einführerweiterung aufgenommen sind und die Andruckscheibe gegen Verdrehung sichern. Durch diese Ausgestaltung wird insbesondere erreicht, dass sich die Andruckscheibe im Rahmenelement beim Drehen der Federscheibe nicht mitdrehen kann, so dass eine Beeinträchtigung des durch die Andruckscheibe "festgeklemmten" Filterblattes sicher ausgeschlossen ist.

Gemäß Anspruch 5 kann des Weiteren vorgesehen sein, dass das Rahmenelement einen umlaufenden, axial zum Wandbereich hin vorstehenden Ringsteg aufweist, der in eine Ringnut des Wandbereichs eingreift und über eine Bajonettverbindung lösbar mit der Ringnut verbunden ist. Durch diese Ausgestaltung der Erfindung lässt sich das Rahmenelement leicht und sicher am Wandbereich befestigen und bei Bedarf, beispielsweise zu Reinigungszwecken, auch abnehmen.

Gemäß Anspruch 6 kann weiter vorgesehen sein, dass die Andruckscheibe und die Federscheibe im Wesentlichen gleich groß sind, aus Metall oder Kunststoff bestehen und innerhalb des Rahmenelements im Wesentlichen in deckungsgleicher Lage ihrer Durchbrüche aufeinander liegen. Durch diese Ausgestaltung ist es insbesondere möglich, die Andruckscheibe und die Federscheibe nach dem Einsetzen des Filterblattes gemeinsam in das Rahmenelement einzusetzen.

Gemäß Anspruch 7 kann vorgesehen sein, dass die Federscheibe im montierten Zustand axial federnd auf der Andruckscheibe aufliegt und durch eine zentrale Achsverbindung konzentrisch drehbar an der Federscheibe zentriert ist. Auch durch diese Ausgestaltung sind die Federscheibe und die Andruckscheibe gemeinsam in das Rahmenelement einsetzbar und lassen sich funktionsgerecht mit diesem verbinden. Durch die zentrale Achsverbindung wird eine Zentrierung zwischen der Andruckscheibe und der Federscheibe erreicht, so dass diese sich beim Einsetzen oder Herausnehmen in bzw. aus dem Rahmenelement nicht radial gegeneinander verschieben können. Durch die axial federnde "Auflage" der Federscheibe auf der Andruckscheibe wird einerseits ein dauernd wirksamer Federdruck mit einer zuverlässigen Abdichtung gewährleistet und andererseits ein zu starker manueller Anpressdruck verhindert. Damit können beim Einsetzen der Federscheibe keine zu großen schädlichen Druckkräfte auf die Andruckscheibe und damit auf das Filterblatt ausgeübt werden.

Weiter kann gemäß Anspruch 8 vorgesehen sein, dass die Federscheibe auf ihrer Oberseite mit wenigstens einem feststehenden Griffteil versehen ist. Durch diese erfindungsgemäße Ausgestaltung ist die Federscheibe äußerst einfach manuell handhabbar. Das Griffteil kann dabei zentral auf der Federscheibe angeordnet sein. Ist auch die gemäß Anspruch 7 beanspruchte gemeinsame Achsverbindung vorgesehen, so sind die Federscheibe und die Andruckscheibe in äußerst einfacher Weise über dieses Griffteil gemeinsam zum Austauschen des Filterblattes handhabbar.

Um beim Einsetzen des Rahmenelementes in die Ringnut des Wandbereichs die erforderliche Kraft bzw. das erforderliche Drehmoment auf das Rahmenelement 4 aufbringen zu können, ist die Ausgestaltung nach Anspruch 9 vorgesehen. Danach ist das ringförmige Rahmenelement oberseitig mit wenigstens zwei sich im Wesentlichen diametral gegenüberliegenden Formschlusselementen versehen. Mit diesen Formschlusselementen ist ein manuell betätigbarer Handhebel drehfest und lösbar formschlüssig in Eingriff bringbar. Damit wird es dem Anwender auf einfache Weise ermöglicht, eine feste, stabile Renkverbindung zwischen dem Rahmenelement und dem Wandbereich herzustellen und diese Verbindung auch wieder zu lösen, falls dies beispielsweise zu Reinigungszwecken erforderlich sein sollte.

Anhand der Zeichnung wird die Erfindung nachfolgend beispielhaft näher erläutert. Es zeigt:
- Fig. 1: eine perspektivische "Unteransicht" eines Behälterdeckels;
- Fig. 2: einen vergrößerten Ausschnitt II der Ringnut des Behälterdeckels aus Fig. 1;
- Fig. 3: eine perspektivische Ansicht eines mit der Ringnut des Behälterdeckels aus Fig. 1 in Eingriff bringbaren Rahmenelements;
- Fig. 4: das Rahmenelement aus Fig. 3 in einer zweiten perspektivischen Ansicht, in welcher der mit der Ringnut des Behälterdeckels in Eingriff bringbare Ringsteg erkennbar ist;
- Fig. 5: einen vergrößerten Ausschnitt V des Rahmenelements aus Fig. 4;
- Fig. 6: eine perspektivische Darstellung einer Federscheibe;
- Fig. 7: eine perspektivische Darstellung einer Andruckscheibe;
- Fig. 8: einen vergrößerten, perspektivischen Ausschnitt der Andruckscheibe aus Fig. 7;
- Fig. 9: ein mit der Federscheibe feststehend verbindbares Griffteil in perspektivischer Darstellung;
- Fig. 10: eine teilweise Ansicht des Behälterdeckels mit vollständig montierter Filtereinheit;
- Fig. 11: einen Teilschnitt XI - XI des Behälterdeckels aus Fig. 10 mit vollständig montierter Filtereinheit;
- Fig. 12: einen vergrößerten Ausschnitt XII des Behälterdeckels aus Fig. 11 im Verbindungsbereich des Rahmenelements zum Behälterdeckel;
- Fig. 13: einen vergrößerten Ausschnitt XIII des Behälterdeckels aus Fig. 11 im Verbindungsbereich des Griffteils zur Federscheibe;
- Fig. 14: eine perspektivische Darstellung eines mit dem Rahmenelement drehfest koppelbaren Handhebels.

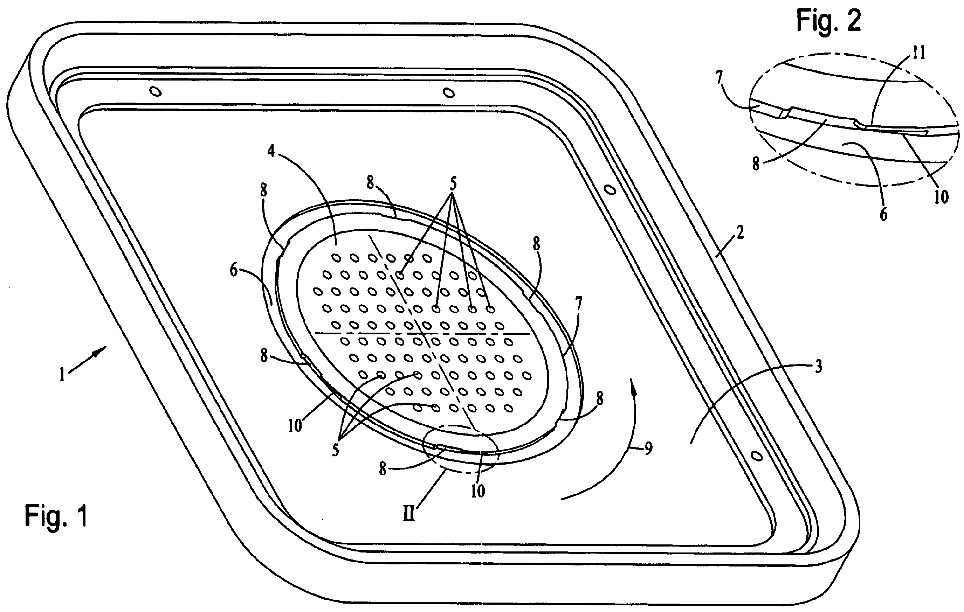

Fig. 1 zeigt beispielhaft eine Ausführungsvariante eines Behälterdeckels 1, welcher eine rechteckig abgerundete Grundform aufweist. Ein solcher Behälterdeckel 1 ist im Betrieb auf ein entsprechendes Behälterunterteil dicht aufsetzbar, welches jedoch in der Zeichnung nicht dargestellt ist. Dieser Behälterdeckel 1 bildet mit diesem Behälterunterteil einen medizinischen Sterilisierbehälter, welcher beispielsweise zum Sterilisieren medizinischer Geräte dient.

Wie aus Fig. 1 ersichtlich ist, weist der Behälterdeckel 1 einen umlaufenden Randsteg 2 auf, welcher ein vorzugsweise ebenes Wandelement 3 des Behälterdeckels 1 umschließt. Des Weiteren ist in der Regel im montierten Zustand des Behälterdeckels 1 auf einem Behälterunterteil eine Dichtung vorgesehen, so dass der Behälterdeckel 1 auf dem Unterteil dicht montierbar ist. Weiter ist aus Fig. 1 ersichtlich, dass das Wandelement 3 einen zentralen Wandbereich 4 aufweist, welcher beim vorliegenden Ausführungsbeispiel mit einer Vielzahl von Gasaustauschöffnungen 5 versehen ist. Im Betrieb findet durch diese Gasaustauschöffnungen beispielsweise bei Erwärmen oder Abkühlen des gesamten Sterilisierbehälters ein Gasaustausch mit der Umgebung statt. In diesem Wandbereich 4 ist innenseitig im Wandelement 3 eine kreisringförmig ausgebildete Ringnut 6 vorgesehen. Diese Ringnut 6 ist im Bereich ihrer umlaufenden Innenkante 7 mit radial nach innen gerichteten Ausnehmungen 8 versehen. Wie insbesondere aus Fig. 2 ersichtlich ist, mündet in diese Ausnehmung 8 jeweils ein Arretierschlitz 10, welcher dementsprechend durch einen Fixiersteg 11 abgedeckt ist. Fig. 2 zeigt diesbezüglich eine vergrößerte Darstellung des Bereiches II aus Fig. 1.

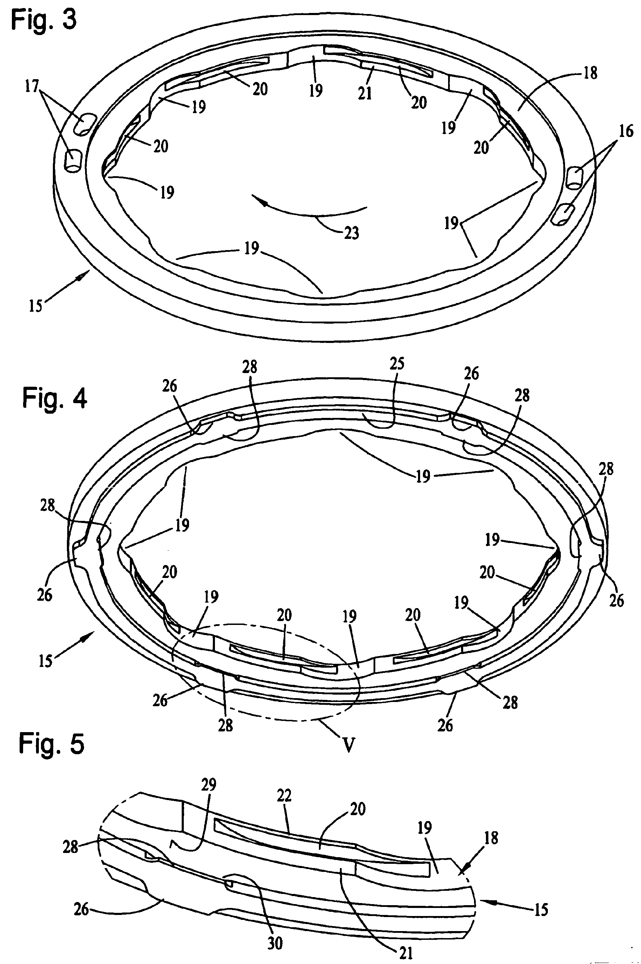

In die Ringnut 6 des Wandelementes 3 bzw. des Wandbereiches 4 ist ein Rahmenelement 15 einsetzbar, welches beispielhaft in den Fig. 3 bis 5 dargestellt ist. Fig. 3 zeigt hierzu eine Art Draufsicht, während Fig. 4 eine Unteransicht dieses Rahmenelementes 15 darstellt.

Aus Fig. 3 ist erkennbar, dass das Rahmenelement 15 kreisringförmig ausgebildet ist und oberseitig jeweils paarweise nebeneinander angeordnete Formschlusselemente 16 und 17 aufweist. Diese Formschlusselemente 16 und 17 liegen sich beim vorliegenden Ausführungsbeispiel im Wesentlichen diametral gegenüber und sind jeweils nach Art eines Langlochs ausgebildet. Diese Formschlusselemente 16 und 17 sind formschlüssig und lösbar mit einem Handhebel in Eingriff bringbar, um das Rahmenelement in einfacher Weise drehend verstellen zu können.

Des Weiteren bildet das Rahmenelement 15 einen radial nach innen vorstehenden, vollständig umlaufenden Haltesteg 18, welcher innenseitig mit gleichmäßig am Umfang verteilt angeordneten Einführungserweiterungen 19 versehen ist. Jede dieser Einführungserweiterungen 19 bildet somit im umlaufenden Haltesteg 18 eine kreisbogenförmig verlaufende radiale Erweiterung, deren Funktion später noch näher erläutert wird.

In jede dieser Einführungserweiterungen 19 mündet eine sich über einen Teilumfang des Rahmenelementes 15 erstreckende Aufnahmenut 20, welche einerseits unterseitig durch ein Stegelement 21 und andererseits oberseitig durch ein weiteres Stegelement 22 begrenzt ist. Diese Einführungserweiterungen 19 bilden zusammen mit den sich in Umfangsrichtung erstreckenden Aufnahmenuten 20 einen Teil einer Renkverbindung zur lösbaren Halterung einer Federscheibe, wie später noch erläutert werden wird.

Fig. 5 zeigt insbesondere hierzu einen vergrößerten Ausschnitt V aus Fig. 4, in welchem eine der Einführungserweiterungen 19 zusammen mit der zugehörigen Aufnahmenut 20 sowie den beiden Stegelementen 21 und 22 näher erkennbar ist.

Es ist leicht vorstellbar, dass beispielsweise eine Scheibe mit radial vorstehenden Verbindungselementen in die Einführungserweiterungen 19 axial einsetzbar und anschließend in Richtung des Pfeils 23 drehbar und somit mit den Aufnahmenuten 20 nach Art einer Renkverbindung feststehend in Eingriff bringbar ist.

Weiter ist aus Fig. 4 ersichtlich, dass das Rahmenelement 15 unterseitig einen axial vorstehenden Ringsteg 25 bildet, mit welchem das Rahmenelement 15 in die Ringnut 6 des Behälterdeckels 1 aus Fig. 1 einsetzbar ist. Dieser Ringsteg 25 bildet beim vorliegenden Ausführungsbeispiel mehrere gleichmäßig am Umfang verteilte, auf einem größeren Durchmesser liegende Zentrierelemente 26, mit welchen der Ringsteg 25 konzentrisch in die Ringnut 6 des Behälterdeckels 1 aus Fig. 1 einsetzbar ist. Bei entsprechender Ausgestaltung der Ringnut 6 können diese Zentrierelemente 26 mit der Ringnut auch eine Art Bajonettverbindung bilden.

Beim vorliegenden Ausführungsbeispiel weist der Ringsteg 25 des Rahmenelementes 15 im Bereich seiner Innenkante 27 mehrere gleichmäßig am Umfang angeordnete Haltestege 28 auf, welche zur feststehenden und lösbaren Halterung des Rahmenelementes 15 in der Ringnut 6 des Wandelementes 3 dienen. Diese Haltestege 28 sind dementsprechend jeweils mit einem der Arretierschlitze 10 der Ringnut 6 des Behälterdeckels 1 formschlüssig in Eingriff bringbar.

Hierzu ist insbesondere aus der vergrößerten Teildarstellung der Fig. 5 erkennbar, dass diese Haltestege 28 einen Abstand zur Unterseite 29 des inneren umlaufenden Haltesteges 18 aufweisen, so dass zwischen dem jeweiligen Haltesteg 28 und dieser Unterseite 29 ein Freiraum 30 zur Aufnahme des jeweils zugeordneten Fixiersteges 11 der Ringnut 6 gebildet wird.

Es ist nunmehr leicht vorstellbar, dass in entsprechender Winkelorientierung das Rahmenelement 15 mit seinen radial nach innen gerichteten Haltestegen 28 seines Ringsteges 25 axial in die Ausnehmungen 8 der Ringnut 6 einsetzbar ist. Dabei gelangen die Haltestege 28 in axialer Richtung in dieselbe Ebene wie die zu Fig. 2 beschriebenen Arretierschlitze 10. Durch Verdrehen des gesamten Rahmenelements 15 in Richtung des Pfeils 9 werden die Haltestege 28 in die Arretierschlitze 10 eingeschoben, so dass zwischen dem Rahmenelement 15 und der Ringnut 6 bzw. dem Behälterdeckel 1 eine Art Bajonettverbindung gebildet wird. Somit ist das Rahmenelement 15 bei entsprechend entgegengesetzter Drehrichtung zum Pfeil 9 aus den Arretierschlitzen 10 wieder lösbar und kann von der Innenseite des Behälterdeckels 1 beispielsweise zu Reinigungszwecken abgenommen werden.

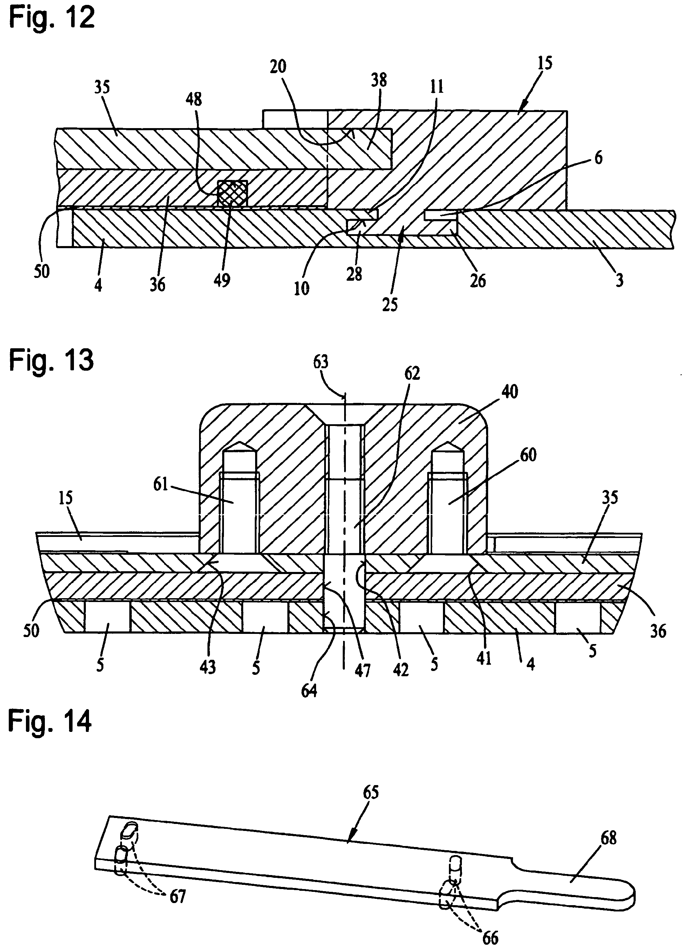

Dieses Rahmenelement 15 dient beim vorliegenden Ausführungsbeispiel zur Arretierung bzw. klemmenden Halterung eines Filterblattes, wie später insbesondere zu den Fig. 12 und 13 noch näher erläutert wird.

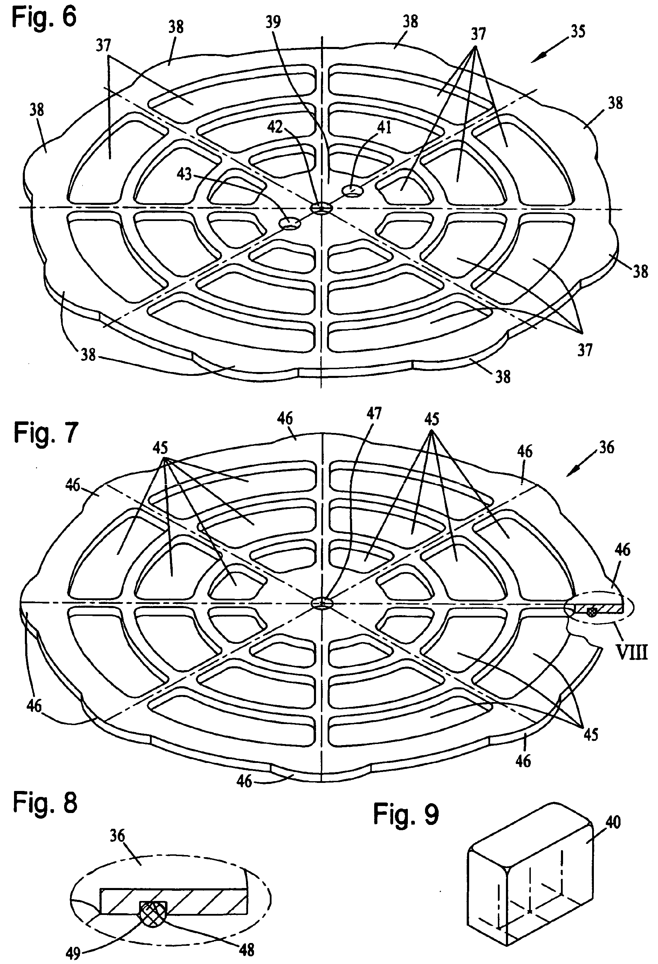

Um ein solches Filterblatt, welches den kompletten Wandbereich 4 mit seinen Gasaustauschöffnungen 5 des Behälterdeckels 1 (Fig. 1 ) vollständig abdeckt, in diesem Wandbereich 4 auswechselbar zu fixieren, sind erfindungsgemäß eine Federscheibe 35 (Fig. 6) sowie eine Andruckscheibe 36 (Fig. 7) vorgesehen.

Wie aus Fig. 6 ersichtlich ist, ist die Federscheibe 35 als Kreisscheibe ausgebildet, wobei deren Außendurchmesser dem Innendurchmesser des Haltesteges 18 des Rahmenelementes 15 angepasst ist, so dass die Federscheibe 35 mit geringem Spiel in den Haltesteg 18 einsetzbar ist. Die Federscheibe 35 weist mehrere großflächige Durchbrüche 37 auf, welche gleichmäßig am Umfang und über den Radius verteilt in der Federscheibe 35 angeordnet sind.

Des Weiteren sind am Außenumfang der Federscheibe 35 mehrere nocken- oder zungenartige Vorsprünge 38 vorgesehen, deren Anzahl und Anordnung am Umfang der Federscheibe 35 der Anzahl und Anordnung der Einführungserweiterungen 19 des Haltesteges 18 des Rahmenelementes 15 entsprechen.

Somit ist die Federscheibe 35 mit ihren Vorsprüngen 38 in den Haltesteg 18 des Rahmenelementes 15 in einer vorbestimmten Winkellage einsetzbar. Im Zentrum der Federscheibe 35 ist beim vorliegenden Ausführungsbeispiel eine Art Halteplatte 39 vorgesehen, welche zur feststehenden Befestigung eines Griffteils 40 (Fig. 9) dient. Dementsprechend weist diese Halteplatte 39 beim vorliegenden Ausführungsbeispiel drei Durchgangsbohrungen 41, 42 und 43 auf, in deren Bereich das Griffteil 40 auf der Halteplatte 39 aufsetzbar ist.

In ähnlicher Baustruktur wie die Federscheibe 35 ist auch die Andruckscheibe 36 aufgebaut. Auch die Andruckscheibe 36 weist mehrere Durchbrüche 45 auf, welche sowohl in Umfangsrichtung als auch in radialer Richtung gleichmäßig in der Andruckscheibe 36 verteilt angeordnet sind. Die Andruckscheibe 36 ist ebenfalls in ihrer Grundstruktur kreisrund ausgebildet und weist an ihrem Außenumfang mehrere gleichmäßig verteilt angeordnete, radial nach außen vorstehende nocken- oder zungenartige Vorsprünge 46 auf. Auch diese Vorsprünge 46 sind in ihrer Anzahl und Anordnung ebenfalls entsprechend der Anordnung und Anzahl der Einführungserweiterungen 19 des Haltesteges 18 des Rahmenelementes 15 am Außenumfang der Andruckscheibe 36 angeordnet. Somit ist auch die Andruckscheibe 36 passend in den Haltesteg 18 des Rahmenelementes 15 einsetzbar, wobei die Vorsprünge 46 in die Einführungserweiterungen 19 passend bzw. mit geringem Spiel eingreifen.

Hierzu sei Stelle angemerkt, dass die die jeweilige Aufnahmenut 20 nach unten hin begrenzenden Stegelemente 21 in ihrer Dicke etwa der Dicke der Andruckscheibe 36 entsprechen. D. h., dass im vollständig eingesetzten Zustand der Andruckscheibe 36 in das am Wandelement 3 fixierten Rahmenelement 15 diese Andruckscheibe 36 durch deren Vorsprünge 46 unverdrehbar durch die die Einführungserweiterungen 19 begrenzenden Stegelemente 21 drehfest gehalten ist.

Weiter ist aus Fig. 7 ersichtlich, dass die Andruckscheibe 36 eine zentrale Durchgangsbohrung 47 aufweist, welche im montierten Zustand koaxial zur mittleren Durchgangsbohrung 42 der Federscheibe 35 verläuft.

Weiter zeigt der vergrößerte Ausschnitt VIII der Andruckscheibe 36 aus Fig. 7 in der Zeichnungsfigur 8, dass unterseitig die Andruckscheibe 36 mit einer Ringnut 48 versehen ist, in welcher ein Dichtungsring 49 umlaufend aufgenommen wird. Mit diesem Dichtungsring 49 ist die Andruckscheibe 36 auf ein im Wandbereich 4 aufgelegtes Filterblatt dicht aufsetzbar.

Vorzugsweise weist die Federscheibe 35 aus Fig. 6 mittig eine leichte, zur Andruckscheibe 36 hin gerichtete Wölbung auf, so dass diese im montierten Zustand federelastisch unter leichter Vorspannung gegen die Andruckscheibe 36 drückt.

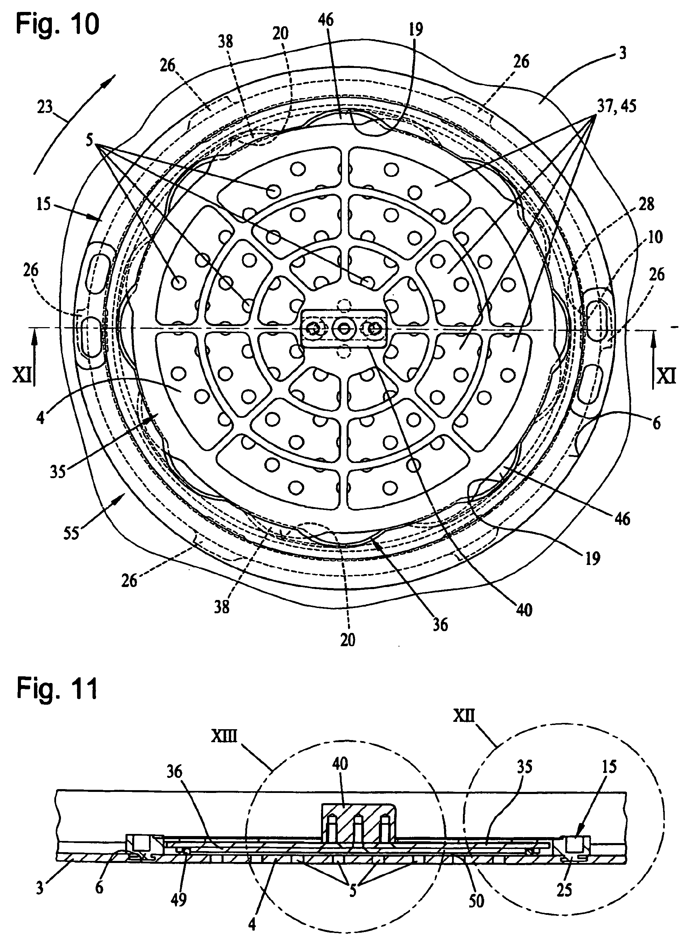

Fig. 10 zeigt eine teilweise Draufsicht auf das Wandelement 3 mit einer vollständig montierten Filtereinheit 55. Es ist erkennbar, dass das Rahmenelement 15 auf dem Wandelement 3 aufgesetzt ist und den Wandbereich 4 vollständig kreisringförmig umgibt. Die radial äußeren Zentrierelemente 26 werden dabei mit äußerst geringem Spiel in der umlaufenden Ringnut 6 des Wandelementes 3 aufgenommen. Andeutungsweise ist des Weiteren aus Fig. 10 erkennbar, dass die radial nach innen vorstehenden Haltestege 28 mit den jeweils zugeordneten Arretierschlitzen 10 der Ringnut 6 in Eingriff stehen.

Weiter ist erkennbar, dass sich die Federscheibe 35 sowie die Andruckscheibe 36 im Rahmenelement 15 befinden. In dieser in Fig. 10 dargestellten montierten Position sind die Durchbrüche 37 und 45 der Federscheibe 35 und der Andruckscheibe 36 deckungsgleich angeordnet. Es ist erkennbar, dass die Vorsprünge 38 der Federscheibe 35 in diesem montierten Zustand in Umfangsrichtung versetzt zu den mit den Einführungserweiterungen 19 in Eingriff stehenden Vorsprüngen 46 der Andruckscheibe 36 angeordnet sind und mit der jeweiligen Aufnahmenut 20 formschlüssig in Eingriff stehen. Die radialen Vorsprünge 46 der Andruckscheibe 36 werden somit in den zugehörigen Einführungserweiterungen 19 des Rahmenelementes 15 passend aufgenommen, so dass die Andruckscheibe 36 unverdrehbar im Rahmenelement angeordnet ist.

Die Federscheibe 35 wurde zur Verriegelung in Richtung des Pfeils 23 gedreht, so dass deren radiale Vorsprünge 38 mit der jeweils zugeordneten Aufnahmenut 20 der Einführungserweiterungen 19 axial feststehend in Eingriff stehen.

Des Weiteren ist aus Fig. 10 erkennbar, dass die Durchbrüche 37 und 45 im Bereich der Gasaustauschöffnungen 5 des Wandbereiches 4 angeordnet sind und diese Gasaustauschöffnungen 5 zumindest annähernd vollständig freigegeben sind.

In Fig. 10 ist das sich zwischen der Andruckscheibe 36 und dem Wandbereich 4 befindende Filterblatt nicht dargestellt, so dass die Gasaustauschöffnungen 5 entsprechend erkennbar sind.

Weiter ist aus Fig. 10 ersichtlich, dass das Griffteil 40 oberseitig auf der Federscheibe 35 montiert ist, so dass die Federscheibe 35 über dieses Griffteil 40 in einfacher Weise zum "Verriegeln" und "Entriegeln" drehbar ist.

Fig. 11 zeigt hierzu eine Schnittdarstellung XI-XI aus Fig. 10. Es ist erkennbar, dass das Griffteil 40 feststehend oberseitig auf der Federscheibe 35 montiert ist. Zwischen der Federscheibe 35 und dem Wandelement 3 bzw. dessen Wandbereich 4 ist die Andruckscheibe 36 angeordnet. Der Dichtungsring 49 liegt oberseitig auf dem andeutungsweise erkennbaren Filterblatt 50 dichtend auf. Dabei deckt das Filterblatt 50 die Gasaustauschöffnungen 5 vollständig ab.

Weiter steht das Rahmenelement 15 mit seinem unteren Ringsteg 25 mit der Ringnut 6 des Wandelementes 3 in Eingriff. Hierzu zeigt Fig. 12 einen vergrößerten Ausschnitt XII aus Fig. 11. Es ist erkennbar, dass das Rahmenelement 15 mit seinen radial nach außen vorstehenden Zentrierelementen 26 des Ringsteges 25 passend in der Ringnut 6 aufgenommen wird. Der radial nach innen vorstehende Haltesteg 28 des umlaufenden Ringsteges 25 steht mit dem entsprechend zugeordneten Arretierschlitz 10 feststehend in Eingriff.

Weiter ist aus Fig. 12 erkennbar, dass das Filterblatt 50 zwischen der Andruckscheibe 36 und dem Wandelement 3 bzw. dem Wandbereich 4 aufgenommen ist. Dabei drückt der Dichtungsring 49 axial gegen das Filterblatt 50, so dass dieses dicht auf dem Wandelement 3 aufliegt. Der radiale Vorsprung 38 der Federscheibe 35 steht dementsprechend mit der zugeordneten Aufnahmenut 20 des Rahmenelementes 15 in Eingriff, so dass die Andruckscheibe 36 mit ihrem Dichtungsring 49 gegen das Filterblatt 50 gedrückt wird. Durch die federelastische Ausgestaltung der Federscheibe 35 und auch des Dichtungsringes 49 ergibt sich somit eine Abdichtung des Wandbereichs 4 mit dem aufgelegten Filterblatt 50 insbesondere gegen das Rahmenelement 15.

Fig. 13 zeigt des Weiteren beispielhaft eine vergrößerte Darstellung XIII aus Fig. 11, aus welcher insbesondere die Art der Befestigung des Griffteils 40 auf der Oberseite der Federscheibe 35 erkennbar ist. Das Griffteil 40 ist mittels zweier Montageschrauben 60 und 61 an der Federscheibe 35 befestigt. Dementsprechend durchragen diese beiden Montageschrauben 60 und 61 die zu Fig. 6 bereits erwähnten Durchgangsbohrungen 41 und 42. Des Weiteren ist in das Griffteil 40 ein Zentrierzapfen 62 eingeschraubt, welcher für die Federscheibe 35 und die Andruckscheibe 36 eine gemeinsame Achsverbindung darstellt und dementsprechend in deren zentrale Durchgangsbohrungen 42 und 47 hineinragt. Dieser Zentrierzapfen 62 ist in seiner Länge derart ausgebildet, dass er des Weiteren in eine zentrale Bohrung 64 des Wandbereichs 4 eingreift und dementsprechend auch das Filterblatt 50 durchragt. Durch diesen Zentrierzapfen 62 werden somit das Filterblatt 50, die Andruckscheibe 36 und die Federscheibe 35 konzentrisch zur Ringnut 6 und somit zu dem mit dieser Ringnut 6 in Eingriff stehenden Ringelement 15 ? drehbar gehalten. Das Filterblatt 50 deckt dabei die Gasaustauschöffnungen 5 im Wandbereich 4 des Wandelementes 3 ab und wird durch die ebenfalls in Fig. 3 erkennbare Andruckscheibe 36 auf der Oberfläche des Wandbereiches 4 gehalten.

Fig. 14 zeigt beispielhaft eine Ausführungsvariante eines Handhebels 65, welcher zur Herstellung und zum Lösen der Bajonettverbindung zwischen dem Rahmenelement 15 und dem Wandelement 3 vorgesehen ist. Hierzu weist der Handhebel 65 unterseitig zwei paarweise angeordnete Eingriffszapfen 66 bzw. 67 auf, welche formschlüssig mit den oberseitig im Rahmenelement 15 angeordneten Formschlusselementen 16 bzw. 17 (Fig. 3) in Eingriff bringbar sind. Weiter ist aus Fig. 14 erkennbar, dass der Handhebel 65 zumindest einseitig ein Griffelement 68 aufweist, durch welches das entsprechend notwendige Drehmoment zur Herstellung der Bajonettverbindung bzw. zum Lösen der Bajonettverbindung aufbringbar ist.

## Patentansprüche

1. Medizinischer, mit einem abnehmbaren Behälterdeckel (1) versehener Sterilisierbehälter, der in einem mit Gasaustauschöffnungen (5) versehenen Wandbereich (4) mit einer Filtereinheit (55) für den Gasaustausch versehen ist, die ein austauschbares, die Gasaustauschöffnungen (5) innenseitig abdeckendes Filterblatt (50) aufweist, das innerhalb eines ringförmig umlaufenden Rahmenelements (15) angeordnet und durch eine mit Durchbrüchen (45) versehene Andruckscheibe (36) gegen die Innenseite des Wandbereichs (4) pressbar gehalten ist, wobei die Andruckscheibe (36) lösbar mit dem Rahmenelement (15) oder dem Wandbereich (4) verbunden ist und eine Ringnut (48) mit einem elastischen Dichtungsring (49) aufweist, der den mit den Gasaustauschöffnungen (5) versehenen Wandbereich (4) gegen das Rahmenelement (15) abdichtet,
**dadurch gekennzeichnet,**
**dass** die Andruckscheibe (36) in das Rahmenelement (15) unverdrehbar einsetzbar und durch eine Federscheibe (35) gegen den Wandbereich (4) pressbar ist und,
**dass** die Federscheibe (35) mit Renkverbindungselementen (38) versehen ist, welche mit Renkverschlusselementen (20) des Rahmenelements (15) in und außer Eingriff bringbar sind und,
**dass** die Federscheibe (35) gegenüber der Andruckscheibe (36) um eine gemeinsame zentrale Drehachse (63) der Andruckscheibe (36) und der Federscheibe (35) drehbar ist.

2. Medizinischer Sterilisierbehälter nach Anspruch 1, **dadurch gekennzeichnet, dass** die Federscheibe (35) mit Durchbrüchen (37) versehen ist, welche nach dem gleichen Flächenmuster ausgebildet und angeordnet sind wie die Durchbrüche (45) der Andruckscheibe (36).

3. Medizinischer Sterilisierbehälter nach Anspruch 1 oder 2, **dadurch gekennzeichnet, dass** die Renkverbindungselemente der Federscheibe (35) aus an ihrem kreisrunden Umfang radial nach außen vorstehenden nocken- oder zungenförmigen Vorsprüngen (38) gebildet sind, die in an der Innenseite des Rahmenelements (15) angeordnete radiale Aufnahmeschlitze oder -nuten (20) eingreifen, welche sich jeweils in einer Drehrichtung (Pfeil 23) an Einführerweiterungen (19) des Rahmenelements (15) anschließen.

4. Medizinischer Sterilisierbehälter nach Anspruch 3, **dadurch gekennzeichnet, dass** sich die Einführerweiterungen (19) über die gesamte Höhe bzw. Dicke des Rahmenelementes (15) erstrecken und dass die Andruckscheibe (36) an ihrem Umfang mit radial nach außen vorstehenden, nocken- oder zungenförmigen Vorsprüngen (46) versehen ist, die jeweils in einer solchen Einführerweiterung (19) aufgenommen sind und die Andruckscheibe (36) gegen Verdrehung sichern.

5. Medizinischer Sterilisierbehälter nach Anspruch 1, **dadurch gekennzeichnet, dass** das Rahmenelement (15) einen umlaufenden, axial zum Wandbereich (4) hin vorstehenden Ringsteg (25) aufweist, der in eine Ringnut (6) des Wandbereichs (4) eingreift und über eine Bajonettverbindung (10, 28) lösbar mit der Ringnut (6) verbunden ist.

6. Medizinischer Sterilisierbehälter nach einem der Ansprüche 1 bis 5, **dadurch gekennzeichnet, dass** die Andruckscheibe (36) und die Federscheibe (35) gleich groß sind, aus Metall oder Kunststoff bestehen und innerhalb des Rahmenelements (15) in deckungsgleicher Lage ihrer Durchbrüche (37 und 45) aufeinander liegen.

7. Medizinischer Sterilisierbehälter nach einem der Ansprüche 1 bis 6, **dadurch gekennzeichnet, dass** die Federscheibe (35) im montierten Zustand axial federnd auf der Andruckscheibe (36) aufliegt und durch eine zentrale Achsverbindung (62) konzentrisch drehbar an der Federscheibe (35) zentriert ist.

8. Medizinischer Sterilisierbehälter nach einem der Ansprüche 1 bis 7, **dadurch gekennzeichnet, dass** die Federscheibe (35) auf ihrer Oberseite mit wenigstens einem feststehenden Griffteil (40) versehen ist.

9. Medizinischer Sterilisierbehälter nach Anspruch 2 oder 3, **dadurch gekennzeichnet, dass** das ringförmige Rahmenelement (15) oberseitig mit wenigstens zwei sich diametral gegenüberliegenden Formschlusselementen (16, 17) versehen ist und dass ein manuell betätigbarer Handhebel (65) vorgesehen ist, welcher formschlüssig mit den Formschlusselementen (16, 17) des Rahmenelementes (15) drehfest und lösbar in Eingriff bringbar ist.

## Claims

1. Medical sterilization container provided with a removable container lid (1), which sterilization container is provided, in a wall area (4) provided with gas-exchange openings (5), with a filter unit (55) for the gas exchange, which filter unit (55) has an exchangeable filter sheet (50) which covers the gas exchange openings (5) on an inner face and is arranged within a ring-shaped circumferential frame element (15) and is held pressably against the inner face of the wall area (4) by a pressure washer (36) provided with apertures (45), wherein the pressure washer (36) is connected releasably to the frame element (15) or to the wall area (4) and has an annular groove (48) with an elastic sealing ring (49), which seals off the wall area (4) provided with the gas exchange openings (5) against the frame element (15),
**characterized**
**in that** the pressure washer (36) can be inserted non-rotatably into the frame element (15) and is pressable against the wall area (4) by a spring washer (35), and
**in that** the spring washer (35) is provided with bayonet connection elements (38) which can be brought into and out of engagement with bayonet closure elements (20) of the frame element (15),
and
**in that** the spring washer (35) is rotatable with respect to the pressure washer (36) about a common central rotation axis (63) of the pressure washer (36) and of the spring washer (35).

2. Medical sterilization container according to Claim 1, **characterized in that** the spring washer (35) is provided with apertures (37), which are designed and arranged according to the same surface pattern as the apertures (45) of the pressure washer (36).

3. Medical sterilization container according to Claim 1 or 2, **characterized in that** the bayonet connection elements of the spring washer (35) are formed by cam-shaped or tongue-shaped projections (38) protruding radially outwards on its circular circumference, which projections engage in radial receiving slits or grooves (20) which are arranged on the inner face of the frame element (15) and which each adjoin insertion expansions (19) of the frame element (15) in a direction of rotation (arrow 23).

4. Medical sterilization container according to Claim 3, **characterized in that** the insertion expansions (19) extend over the entire height or thickness of the frame element (15), and **in that** the pressure washer (36) is provided on its circumference with radially outwardly protruding, cam-shaped or tongue-shaped projections (46), which are each received in one such insertion expansion (19) and secure the pressure washer (36) against rotation.

5. Medical sterilization container according to Claim 1, **characterized in that** the frame element (15) has a circumferential annular web (25) which protrudes axially towards the wall area (4) and which engages in an annular groove (6) of the wall area (4) and is connected releasably to the annular groove (6) via a bayonet connection (10, 28).

6. Medical sterilization container according to one of Claims 1 to 5, **characterized in that** the pressure washer (36) and the spring washer (35) are the same size, are made of metal or plastic and lie on each other inside the frame element (15) with their apertures (37 and 45) in a congruent position.

7. Medical sterilization container according to one of Claims 1 to 6, **characterized in that** the spring washer (35) in the mounted state bears axially resiliently on the pressure washer (36) and is centred concentrically rotatably on the spring washer (35) by a central axial connection (62).

8. Medical sterilization container according to one of Claims 1 to 7, **characterized in that** the spring washer (35) is provided, on its top face, with at least one fixed grip part (40).

9. Medical sterilization container according to Claim 2 or 3, **characterized in that** the annular frame element (15) is provided, on a top face, with at least two diametrically opposite form-fit elements (16, 17), and **in that** a manually actuatable hand lever (65) is provided which can be brought into rotationally fixed and releasable form-fit engagement with the form-fit elements (16, 17) of the frame element (15).

## Revendications

1. Récipient médical de stérilisation, pourvu d'un couvercle de récipient amovible (1), qui est muni, dans une région de paroi (4) dotée d'ouvertures d'échange de gaz (5), d'une unité de filtre (55) pour l'échange de gaz, qui présente une feuille de filtre (50) échangeable, recouvrant les ouvertures d'échange de gaz (5) du côté intérieur, qui est disposée à l'intérieur d'un élément de cadre périphérique en forme d'anneau (15) et qui est maintenue en compression contre le côté intérieur de la région de paroi (4) par un disque de pression (36) muni de passages (45), dans lequel le disque de pression (36) est relié de façon séparable à l'élément de cadre (15) ou à la région de paroi (4) et présente une rainure annulaire (48) avec un anneau d'étanchéité élastique (49), qui assure l'étanchéité entre la région de paroi (4) dotée des ouvertures d'échange de gaz (5) et l'élément de cadre (15), **caractérisé en ce que** le disque de pression (36) peut être inséré sans rotation dans l'élément de cadre (15) et peut être pressé par une rondelle élastique (35) contre la région de paroi (4), et **en ce que** la rondelle élastique (35) est munie d'éléments d'assemblage à baïonnette (38), qui peuvent être mis en prise avec des éléments de joint à baïonnette (20) de l'élément de cadre (15) et dégagés de ceux-ci, et **en ce que** la rondelle élastique (35) peut tourner par rapport au disque de pression (36) autour d'un axe de rotation central commun (63) du disque de pression (36) et de la rondelle élastique (35).

2. Récipient médical de stérilisation selon la revendication 1, **caractérisé en ce que** la rondelle élastique (35) est munie de passages (37), qui sont configurés et disposés avec le même motif de surface que les passages (45) du disque de pression (36).

3. Récipient médical de stérilisation selon la revendication 1 ou 2, **caractérisé en ce que** les éléments d'assemblage à baïonnette de la rondelle élastique (35) sont formés par des protubérances (38) en forme de cames ou de languettes saillantes radialement vers l'extérieur à leur périphérie circulaire, qui s'engagent dans des fentes ou des rainures de réception radiales (20) disposées sur le côté intérieur de l'élément de cadre (15), lesquelles se raccordent respectivement dans un sens de rotation (flèche 23) à des élargissements d'introduction (19) de l'élément de cadre (15).

4. Récipient médical de stérilisation selon la revendication 3, **caractérisé en ce que** les élargissements d'introduction (19) s'étendent sur toute la hauteur ou l'épaisseur de l'élément de cadre (15) et **en ce que** le disque de pression (36) est muni à sa périphérie de protubérances en forme de cames ou de languettes (46), saillantes radialement vers l'extérieur, qui sont respectivement logées dans un de ces élargissements d'introduction (19) et qui bloquent le disque de pression (36) contre une rotation.

5. Récipient médical de stérilisation selon la revendication 1, **caractérisé en ce que** l'élément de cadre (15) présente une nervure annulaire périphérique (25), saillante axialement vers la région de paroi (4), qui s'engage dans une rainure annulaire (6) de la région de paroi (4) et qui est reliée à la rainure annulaire (6) de façon séparable par un assemblage à baïonnette (10, 28).

6. Récipient médical de stérilisation selon l'une quelconque des revendications 1 à 5, **caractérisé en ce que** le disque de pression (36) et la rondelle élastique (35) sont de la même grandeur, se composent de métal ou de matière plastique et sont situés l'un sur l'autre à l'intérieur de l'élément de cadre (15) avec recouvrement complet de leurs passages (37 et 45).

7. Récipient médical de stérilisation selon l'une quelconque des revendications 1 à 6, **caractérisé en ce que** la rondelle élastique (35) repose à l'état monté avec élasticité axiale sur le disque de pression (36) et est centrée par une liaison axiale centrale (62) de façon concentriquement rotative sur la rondelle élastique (35).

8. Récipient médical de stérilisation selon l'une quelconque des revendications 1 à 7, **caractérisé en ce que** la rondelle élastique (35) est munie sur son côté supérieur d'au moins une partie de saisie fixe (40).

9. Récipient médical de stérilisation selon la revendication 2 ou 3, **caractérisé en ce que** l'élément de cadre annulaire (15) est muni sur son côté supérieur d'au moins deux éléments d'emboîtement (16, 17) diamétralement opposés l'un à l'autre et **en ce qu'**il est prévu un levier actionnable manuellement (65), qui peut être amené en prise, sans rotation et de façon séparable, par emboîtement avec les éléments d'emboîtement (16, 17) de l'élément de cadre (15).
